# EUROPEAN PATENT SPECIFICATION

(11) **EP 3 362 018 B1**
(45) Date of publication and mention of the grant of the patent: **08.07.2020**
(21) Application number: 15906324.7
(22) Date of filing: 16.10.2015
(51) Int. Cl.: A61F 13/551, B65D 65/14, B65D 75/20

(54) **PACKAGING UNIT FOR A DISPOSABLE HYGIENE ARTICLE AND METHOD OF FORMING THE PACKAGING UNIT**
VERPACKUNGSEINHEIT FÜR EINEN EINWEGHYGIENEARTIKEL UND VERFAHREN ZUR HERSTELLUNG DER VERPACKUNGSEINHEIT
UNITÉ D'EMBALLAGE POUR UN ARTICLE D'HYGIÈNE JETABLE ET PROCÉDÉ DE FORMATION DE L'UNITÉ D'EMBALLAGE

(43) Date of publication of application: 22.08.2018
(73) Proprietor: Essity Hygiene and Health Aktiebolag, 405 03 Göteborg (SE)
(72) Inventor: PETRO BEDOYA, Natalia Maria, Medellin (CO); SANCHEZ VALDES, Juan Sebastian, Medellín (CO)
(74) Representative: Nederlandsch Octrooibureau
(86) International application number: PCT/SE2015/051102
(87) International publication number: WO 2017/065661

(56) References cited:
- WO-A1-88/10219
- WO-A1-99/52485
- WO-A1-2004/069684
- WO-A1-2013/031647
- WO-A1-2015/060755
- WO-A2-2015/002332
- CN-A- 1 895 197

## Description

### TECHNICAL FIELD

The present invention relates to a packaging unit for disposable hygiene articles and to a method of forming a packaging unit for disposable hygiene articles from a sheet of material.

### BACKGROUND OF THE INVENTION

Disposable hygiene articles, such as sanitary napkins and panty liners, can be packaged individually in a wrap of thin plastic film material. Individual packages enable hygienic carrying of the disposable hygiene articles for example in a bag or pocket. The package has a purpose of protecting the disposable hygienic article against contamination, but may also have other functions. For example, the package may function to protect an adhesive layer which is necessary to affix the article to a garment. The adhesive layer is applied on the garment side of the disposable hygienic article. When the package is opened and the wrap removed the adhesive layer is exposed and the article is ready to be affixed to a garment. An additional release liner may be attached to the package wrap, but when the wrap is removed, also the release liner is removed or the release liner can be removed separately. To keep out for example particulate contaminants, the edges of the individual packages are sealed for example by means of ultrasonic welding or heat.

The wrap of the packaging may be used both as a means for packaging an unused article and for disposal of a used article. It is desirable that used disposable hygiene articles can be disposed of discretely and hygienically, especially when the user lacks possibility to discard the used article immediately after the used article has been replaced. Problem may arise for example when there is no waste bin available in the toilet area and the used disposable article needs to be initially put in a bag or pocket. In that case it is required that the wrap tightly seals the used disposable article in order to avoid staining and odour.

A conventional type of packaging unit intended for packaging single articles comprises an elongated rectangular sheet of material which is folded to form a bag-like package with a folded-over lid, also called e-folding. These known packaging units are welded along the edges by means of heat or ultrasound, and are opened by tearing along the welded seams at the edges, so that the package may be unfolded to expose the contents of the package. The main drawback with welded packaging units of this kind is that they cannot be used satisfactorily as disposal bags, since once opened, the packages cannot be resealed and therefore do not allow a used article to be packaged in an acceptable manner with regard to hygiene. Furthermore, the unfolded packaging sheet has an aesthetic appearance, since the broken welds are often uneven and frilly.

In the prior art there have been several suggestions for packaging disposable hygiene articles in individual packages. For example, WO 88/10219 discloses a packaging unit which is formed by folding an elongated sheet of material provided along the mutually parallel edges thereof with a continuous or broken narrow coating of pressure-sensitive adhesive by means of which the container part is held together, with adhesive surfaces lying against and bonding to adhesive surfaces. The packaging unit further comprises a lid coated at least partially with a pressure-sensitive adhesive so as to enable the lid to be refastened to the outside of the container part. Such a packaging unit thus has strongly sealed side seams where the adhesive-coated surfaces are in contact with each other, and a resealable lid providing a tightly sealed disposal package. However, it is difficult to open the package such that equal forces are provided on the sealed side seams, whereby there may be a risk that the wrap sticks to the adhesive and thus the wrap will be broken. This decreases the area for positioning of a used item, which thus has to be inserted into the rather limited container part. This may be a great disadvantage, since the used hygiene article often becomes more bulky than before use, making it difficult to insert it into the container part.

US H1454 discloses a recloseable sheet for packaging and disposal of hygiene articles. The sheet comprises edge adhesive strips positioned such that when the sheet is folded the adhesive-coated areas do not come into contact with each other. In order to serve its purpose, the folding of the sheet has to be initiated from the adhesive-free transverse edge of the sheet. If the folding is initiated from the adhesive-coated transverse edge, two layers of adhesive will overlap each other, and the adhesive-free transverse edge will be positioned at the outer position of the package, i.e. the package will not be tightly sealed.

Further document WO2012/102071 A1 discloses an absorbent article packaging which is provided with an absorbent article having a liquid-permeable surface sheet, a liquid-impermeable backing sheet, and an absorbent, and a wrapping sheet that is folded in the longitudinal direction of the absorbent article and individually wraps the absorbent article. An adhesive material is disposed on the two edges of the wrapping sheet in the width direction that is orthogonal to the longitudinal direction. The adhesive material glues the wrapping sheet to itself where the wrapping sheet is disposed opposite itself when folded. However, there is a risk for contamination of the absorbent article, since after folding of the package the opening end of the package is not sealed and the absorbent article is located in close proximity of the opening end. Also, the opening end requires a separate tab to enable opening of the package. Separate tabs are not desired, since they complicate the manufacturing process.

Thus, even though there are prior art solutions for individual packages for disposable hygienic articles, there is still a need for a packaging unit which can be used both for packaging a new hygiene article and for hygienic keeping and discarding of the used hygiene article, and which is easy to open and protects the hygienic article from contamination.

### SUMMARY OF THE INVENTION

The object of the present invention is to provide a packaging unit which can be used both for packaging a new hygiene article and for safe and hygienic disposing of the used hygiene article.

Especially, it is an object of the present invention to provide a packaging unit that is easy to open and less complicated to produce than prior art packaging units.

It is also an object of the present invention to provide for a possibility of forming a tight package both for a new and a used hygiene articles, thus keeping the new article sanitary and clean prior to use, and eliminating the risk of staining and odour when a used article is packaged.

Further, it is an object of the invention to provide a packaging unit that is easy to unfold and reseal, and is aesthetically appealing.

The objects above are attained with a packaging unit for disposable hygiene articles and a method of forming a packaging unit according to the invention and as defined in the appended claims.

According to a first aspect the packaging unit comprises:
- an elongated sheet of packaging material having an inner surface and an outer surface and the elongated sheet comprising:
   - a first longitudinal end zone and a second longitudinal end zone, wherein the longitudinal end zones extend in a direction transverse to a longitudinal direction of the elongated sheet, and
   - two side edge zones which extend in the longitudinal direction of the elongated sheet, and
   - the side edge zones and the first longitudinal end zone comprising a resealable adhesive coating string along the length of the side edge zones and the first longitudinal end zone, respectively, and wherein
- the unit further comprises an elongated disposable hygiene article extending in a longitudinal direction of the elongated sheet placed on the inner surface of the sheet such that it fits within an area limited by the side edge zones and the first longitudinal end zone.

The unit is formed by folding the elongated sheet and the disposable hygiene article, wherein the unit has a first folding axis and a second folding axis. Each folding axis extends in a direction transverse to the longitudinal direction of the elongated sheet. The first folding axis is arranged between a third region in the packaging unit and a second region in the packaging unit and the second folding axis is arranged between the second region and a first region of the packaging unit. The first region is arranged to form a lid for the packaging unit after folding. The first longitudinal end zone is comprised in the first region and comprises at least two adjacent resealable adhesive coating strings that extend along the length of the first longitudinal end zone and the strings being arranged such that a central adhesive free area is formed. The two resealable adhesive coating strings are adjacent in the direction transverse to the longitudinal direction of the elongated sheet.

The adhesive free area or region in the first longitudinal zone forms a centrally located gripping gap in the first region which after folding forms a lid for the unit. Thus, it will be easy to open the packaging unit since equal pulling forces can be applied to the resealable adhesive coating strings located in the first end zone and in the side edge zones whereby neat opening of the package will be obtained. Thus, the packaging material will not brake due to the pulling forces and therefore, the elongated sheet will be possible to use for discarding of a used hygiene article. Due to the resealable adhesive coating strings, it will be easy to obtain hygienic discharging of a used article. Further, since the first end zone includes resealable adhesive coating strings, the disposable hygiene product can be protected against contamination before use. Further, the central adhesive free area together with the resealable adhesive eliminates a need for additional tabs or elements in the packaging unit, which will complicate the product and the production process thereof.

The disposable hygiene article is suitably placed on the inner surface of the sheet at a predetermined distance from the resealable adhesive coating strings in the first longitudinal end zone, the predetermined distance being in the direction of a central axis of the unit. The predetermined distance should be sufficient to prevent the article from coming into contact with the resealable adhesive coating strings. The distance could be for example from 0.5 mm to 50 mm, but could be more or less. In addition to preventing the article from coming into contact with the resealable adhesive coating strings, the article can be protected from being contaminated after folding of the unit the longer the distance to the resealable adhesive coating strings is.

The article preferably has an end portion having a predetermined length that extends beyond the second longitudinal end zone. The end portion may function as a gripping portion when unfolding the package. Also, in this way it is possible to further increase the distance between the article and the resealable adhesive coating strings in the first longitudinal end zone, since the article can be positioned beyond the second longitudinal end zone. Thereby the risk for contamination of the article may be further decreased.

According to one embodiment, the unit is arranged to be folded along a first folding axis such that the third region is pressed against the second region and the end portion that extends beyond the second longitudinal end zone is pressed against the first region. The unit is further arranged to be folded along a second folding axis such that the first region together with the end portion are pressed against the outer surface of the elongated sheet in the third region, whereby the end portion forms a gripping portion when the unit is opened. When the end portion is also folded, the function as a gripping portion will be more visualized and thus it will be easier for the user to utilize the end portion as a gripping portion.

The end portion that forms a gripping portion makes the unit easy to open since the end portion is centrally located on the elongated sheet whereby equal pulling forces, when the unit is opened, can be applied to the resealable adhesive coating strings located in the side edge zones. Therefore, neat opening of the package will be obtained and the packaging material will not brake due to the pulling forces. Due to the equal pulling forces applied on the resealable adhesive coating strings or regions, it will be possible to use adhesive-to-adhesive contact when forming the folded packaging unit. This is unexpected and it has not been suggested in the prior art since it has been difficult to open packaging units with prior art structural solutions. However, by using the hygiene product in itself to provide a gripping tab, simple structure enabling easy opening without the use of additional components or elements can be provided. This is a huge advantage and makes the product easy to manufacture and more environmentally friendly. Also, due to the resealable adhesive coating strings, it will be easy to obtain hygienic discharging of a used article.

The packaging unit that includes both the resealable adhesive coating strings in the first longitudinal end zone arranged so that there is a central adhesive free area that forms a gripping portion or gap when the unit is opened and the end portion that forms a gripping portion when the unit is opened provides a packaging unit that is easy to open. The centrally located gripping gap in the lid of the unit facilitates the opening of the packaging unit in a first opening step. Equal pulling forces can be applied to the resealable adhesive coating strings located in the first end zone and in the side edge zones in all regions of the packaging unit whereby neat opening of the package will be obtained. Also, since the first end zone includes resealable adhesive coating strings, the disposable hygiene product can be protected against contamination before use. Further, the central adhesive free area together with the resealable adhesive eliminates a need for additional tabs or elements in the packaging unit, which will complicate the product and the production process thereof.

The end portion comprises a second end edge of the article. The second end edge is preferably rounded, i.e. it has a rounded shape. This will further facilitate the gripping of the end portion when opening the unit while the hygiene article will be comfortable to wear.

The disposable hygiene article may comprise a release liner releasably fastened to a garment facing side of the article by means of an adhesive and wherein the release liner is fastened to the inner surface of the elongated sheet by means of adhesive. The release liner protects the adhesive regions on the garment-facing side of the hygiene article while it also provides for a more robust structure for the packaging unit.

The release liner is preferably fastened to the inner surface of the elongated sheet by means of separate adhesive regions placed in the first, second and third region, respectively. In this way, easy disposal of the release liner can be provided, since the release liner will easily stay attached to the elongated sheet when the hygiene article is removed for use.

The disposable hygiene article may alternatively be fastened directly to the inner surface of the elongated sheet by means of adhesive. Thus, no release liner is included, and the weight and the mechanical structure of the packaging unit are further simplified. However, in this case the inner surface of the elongated sheet is preferably coated with a silicone-based coating in the areas where the hygiene article is placed and wherein the areas where the resealable adhesive is coated as resealable adhesive coating strings are free of the silicone-based coating. The silicone-based coating improves the mechanical properties of the packaging material and makes it easy to remove the hygiene article from the elongated sheet.

The elongated sheet has suitably a rectangular shape. By rectangular is meant substantially rectangular with manufacturing tolerances. Rectangular shape makes the unit easy to manufacture.

The elongated sheet of packaging material may be a plastic film, such as a polyethylene film, nonwoven or plastic laminate. Preferably the basis weight is of from 20 to 30 g/m². Thus, a light-weight product with sufficient stability may be provided.

The second longitudinal end zone between the resealable adhesive coating strings in the side edge zones is preferably adhesive-free. By "between the resealable adhesive coating strings in the side edge zones" it is meant that the resealable adhesive coating strings in the side edge zones may extend to the second longitudinal end zone and all the way to the second end edge, whereby there are adhesive portions in the second longitudinal end zone, but the area between these adhesive portions is adhesive-free. In this way the opening of the unit is further facilitated, and it is possible to place the disposable hygiene article such that the end portion extends beyond the second longitudinal end zone

It is further advantageous when an adhesive free gap is formed between the resealable adhesive coating strings extending along the side edge zones of the sheet and the resealable adhesive coating strings extending along the first longitudinal edge zone. The gaps avoid the adhesive or glue overlapping between the resealable adhesive coating strings which could result in a need for excessive opening strength. In this way the opening of the product is further facilitated and evacuation of air from the package is enabled in an easy way.

The resealable adhesive may be a pressure sensitive hotmelt adhesive. Therefore, the unit can be easily reopened and reclosed.

The sheet may be opaque and/or comprises print. In this way it is possible to improve the aesthetical appearance of the unit and it will be possible to mask the used hygiene article when it is wrapped into the sheet after use.

The present invention also relates to a method of forming a packaging unit for disposable hygiene articles. The unit comprises an elongated sheet of packaging material and a disposable hygiene article.

The method according to a first aspect comprises the steps of:
- providing an elongated sheet of packaging material having an inner surface and an outer surface, a first longitudinal end zone and a second longitudinal end zone, wherein the longitudinal end zones extend in a direction transverse to a longitudinal direction of the elongated sheet, and two side edge zones which extend in the longitudinal direction of the elongated sheet,
- attaching an elongated disposable hygiene article extending in the longitudinal direction of the elongated sheet and placing the article on the inner surface of the sheet so that it fits within the area limited by the side edge zones and the first longitudinal end zone,

- coating resealable adhesive strings along the lengths of the side edge zones and the first longitudinal end zone, and forming a central adhesive free area between the resealable adhesive coating strings in the first longitudinal end zone,
- providing a first folding axis and a second folding axis in a direction transverse to the longitudinal direction of the unit which divide the unit into a first region, second region and a third region,
- folding the elongated sheet with the disposable hygiene article along the first folding axis inwards, such that the inner surface of the elongated sheet and the resealable adhesive coating strings in the third region and the second region are pressed against each other,
- folding the elongated sheet with the disposable hygiene article along a second folding axis inwards, such that the outer surface of the sheet in the third region is pressed against the resealable adhesive coating strings in the first region and the inner surface of the sheet in the first region, and
- closing the packaging unit by applying pressure.

According to a further aspect of the invention, in the method, when folding the elongated sheet of the packaging material and the disposable hygiene article along the first folding axis inwards such that the inner surface of the elongated sheet and the resealable adhesive coating strings in the side edge zones are pressed against each other, and when the third region is pressed against the outer surface of the sheet in the second region, an end portion extending beyond a second end zone of the sheet is pressed against the first region. Further, when folding the elongated sheet with the disposable hygiene article along a second folding axis inwards such that the outer surface of the sheet in the third region is pressed against the resealable adhesive coating strings in the first region and the inner surface of the sheet in the first region, the end portion is pressed towards the first folding axis and therefore forms gripping portion when the unit is opened.

The method may further comprise attaching a release liner to the disposable hygiene article by means of adhesive regions, and by attaching the disposable hygiene article to the elongated sheet by means of adhesive regions located in the first, second and third region, respectively.

Further features and advantages with the present invention are defined in the following detailed description with reference to the appended drawings.

### BRIEF DESCRIPTION OF THE DRAWINGS

Fig. 1a shows schematically a packaging unit according to an embodiment of the present invention in an open state with an unused disposable hygiene article. The view is straight from the above and layers and parts of the product locating underneath the tope sheet are marked with dotted lines.
Fig. 1b shows schematically a packaging unit according to a second embodiment of the present invention in an open state with an unused disposable hygiene article. The view is straight from the above and layers and parts of the product locating underneath the tope sheet are marked with dotted lines.
Fig. 2 shows a partly folded product of Fig. 1b in perspective view.
Fig. 3a-3c schematically show folding procedure for the packaging unit shown in Fig. 1a from a side view.
Fig. 4a-4e schematically show folding procedure for the packaging unit shown in Fig. 1b from a side view.

### DETAILED DESCRIPTION

As mentioned above the features of the packaging unit for disposable hygiene articles according to the present invention facilitate opening of the packaging unit. By having a structure in which equal forces can be applied to the resealable adhesive coating strings simultaneously a neat opening without breaking of the packaging unit can be provided. This is a huge advantage compared to prior art solutions in which complicated adhesive string patterns have been used to avoid adhesive to adhesive contact surfaces or in which separate patches or other elements have been used to enable neat opening of the packaging unit. In the following description the components of the packaging unit are described more in detail. The disposable hygiene article may be for example a sanitary napkin or a panty liner. Such products are well known in the art and therefore not described more in detail herein. Any disposable hygiene article could be used in the packaging unit of the present invention.

The elongated sheet of packaging material used in a packaging unit according to the present invention can be a single-ply or laminate material. By the term "single ply" is meant a packaging unit comprising a single ply of a coherent material. Examples of a single ply packaging unit may be a plastic film, such as a polyethylene film, a nonwoven material, a metallic foil or the like. A single ply material may be a non-homogenous material, such as a plastic film material comprising integrated layers, or a nonwoven material having varying fibre composition in different parts of the material. A single ply material as used herein does not comprise materials having separable layers. The single-ply material may be of polyolefin that may comprise or be based on polyethylene and/or polypropylene. The material of polyethylene and/or polypropylene may have a basis weight of 14 to 30 g/m², suitably 20-30g/m².

By the term "laminate" is meant a material comprising at least two united separable plies of material that can be the same or different. In the context of the present invention, the laminate may for example be constituted of two separable plies of plastic film, a film and nonwoven, two plies of nonwoven or the like.

The plastic film in the laminate or single-ply may be olefin-based, such as polyethylene or polypropylene film, of it may be any other known plastic packaging film material in the market, such as polyester-based, nylon, polyvinyl chloride, biodegradable plastic, etc.

The sheet of the packaging material has an elongated shape and has an inner surface and an outer surface. The geometrical shape of the sheet can vary depending on the type of the hygiene article to be packaged. The sheet may be circular, triangular, square, rectangular, or any other shape suitable for the hygiene article to be packaged. It is desirable, however, that the sheet has at least one symmetry axis. The sheet is suitably rectangular meaning that it has four sides and opposing sides have substantially equal length. Also the rectangle is suitably equiangular, each angle being 90°.

The sides of the sheet have edge zones. By the term "edge zone" is meant the portion of the sheet/packaging unit adjacent to the edges of the packaging unit. The width of an edge zone may be varied. The edge zones may be at least partially coated or covered with adhesive coating. In order to avoid that the edges of the sheet curl up or crinkle when the packaging unit is open, the resealable adhesive coating strings provided on the outer edge portions may be positioned at a distance from the longitudinal edges. This distance may be between 0.5-10 mm, preferably between 2-5 mm. Thus the edge zone may for example have a width that varies from 1.5-20 mm.

The elongated sheet has an inner surface and an outer surface. As used herein, the term "inner surface" refers to the surface of the sheet facing the product positioned inside the packaging unit, and the term "outer surface" refers to the surface opposite to the inner surface, i.e. the surface facing the ambient.

The elongated sheet may have a length that corresponds to the length of the disposable hygiene article, or it may be shorter and longer, preferably from about 0-15% shorter or longer than the disposable hygiene article, e.g. 0-15 mm shorter or longer. The elongated sheet is wider than the disposable hygiene article, and can be from about 20-70% wider the disposable hygiene article, e.g. 10-50 mm wider. For example, if the disposable hygiene article is a sanitary napkin having a length of 230 mm the elongated sheet may have a length in a longitudinal direction L of the unit of from 190 to 265 mm, suitably for example 230 mm. If the width of the same sanitary napkin, measured in a direction perpendicular to the longitudinal direction of the unit is 75 mm, the width of the elongated sheet may be from 90 to 128 mm, suitably for example 115 mm.

The disposable hygiene article is preferably placed on the inner surface of the elongated sheet at a predetermined distance from the resealable adhesive coating strings in the first longitudinal zone. This distance can be for example from 0.5 to 20% of the length of the elongated sheet, such as from 0.5 mm to 50 mm, depending on the dimensions of the elongated sheet and the disposable hygiene article.

Further, the disposable hygiene article is preferably placed on the inner surface of the elongated sheet such that an end portion having a predetermined length extends beyond the second longitudinal edge. The end portion may have a rounded shape in the second end edge. The longest extension or length of the a predetermined length extending beyond the second longitudinal edge may correspond to the distance the disposable hygiene article is placed from the resealable adhesive coating strings in the first longitudinal zone, or the longest length may be shorter or longer. The longest or maximum length of the end portion in the longitudinal direction may be for example from 0.5 to 20% of the length of the elongated sheet, such as from 0.5 mm to 50 mm, depending on the dimensions of the elongated sheet and the disposable hygiene article.

The width of the resealable adhesive coating strings or edge portions may be varied depending on the adhesive strength desired. By width is meant the extension of the string in a direction perpendicular to the longitudinal extension of the string, for example in case of a string that extends along the length of a side edge zone, the width is measured in the direction perpendicular to the extension along the length of the side edge zone. The wider the string is the stronger the sealing is. The width of the strings may be same or different in the different regions. For example, the width of the resealable adhesive coating string or strings in the first longitudinal end zone and the side edge zones may be from 1-15 mm. Suitably, the resealable adhesive coating strings are narrower in the first longitudinal end zone than in the side end zones, whereby the opening of the lid of the package is further facilitated. For example, the width of the resealable adhesive coating strings in the first longitudinal end zone may be from 1-3 mm, preferably from 1.5-2.5 mm. The width of the resealable adhesive coating strings in the side edge zones may be from 2-10 mm, preferably 6-8 mm.

The length of the resealable adhesive coating strings in each edge zone may be equal to the length of each edge zone, or may be shorter than the length of each edge zone. The length is measured in the direction of the longitudinal extension of each edge zone.

One of the most common folding patterns for individually wrapped hygiene products is so called e-folding. In this case the sheet has two folding axes, dividing the sheet into a first region, a second region and a third region. The packaging unit may then be formed, wherein the sheet is folded along the folding axes with the first, second and third regions in an overlapping configuration. The folding procedure is illustrated below with reference to the appended drawings.

If desired, the sheet for forming a packaging unit according to the present invention may be opaque in order to disguise the contents of the packaging unit, which is particularly important if the used article wrapped into the packaging unit of the present invention cannot be disposed immediately after replacement. Further, the sheet may comprise print, which may be beneficial for attracting the user's attention.

The sheet of material forming a packaging unit according to the present invention may comprise an odour-inhibiting or odour-neutralising substance. Such a substance may be applied in any suitable manner known to the person skilled in the art, e.g. as coating, activatable microcapsules, impregnated patches or the like.

It is conceivable that the sheet for forming a packaging unit according to the present invention may be stretchable or expandable, which may be advantageous if the hygiene article is greatly deformed during use, and may thus be difficult to wrap without deforming the packaging unit.

A suitable adhesive to be used with the packaging unit of the present invention is resealable adhesive. By a "resealable adhesive" as used herein is meant adhesive that provides that an object may be attached to a surface using such an adhesive and subsequently detached from that surface causing minor or no disruptions of the object and the surface. Furthermore, an object that has been peeled off may also be reattached to a surface again.

A suitable adhesive may be a pressure-sensitive hotmelt adhesive having an unlimited open time, meaning that the adhesive can bond to another substrate at any time. The pressure-sensitive adhesive used with the packaging unit is one which has a very high self-adhesion but which can be readily separated or released from other materials, such as plastic materials or paper which has been treated with a release agent. A major advantage of the packaging unit according to the present invention is that it can be completely unfolded when a new hygiene article is about to be taken out. In contrast thereto, prior art packages having adhesively sealed edges with adhesive-coated edge portions being in contact with each other has too high adhesive strength of the adhesively sealed edges, and any attempt to completely unfold the package would lead to tearing and breakage of the packaging unit, making it unusable for discrete and hygienic disposal of the used article.

The adhesive used in the present invention may be a pressure-sensitive hotmelt adhesive, such as Lunatack® D656 BD 19 available from H. B. Fuller.

The packaging unit may include a release liner having a sheet facing surface and a surface facing the disposable hygiene article. The purpose of the release liner is to cover adhesive regions provided on a garment facing side of the disposable hygiene article that are used to attach the disposable hygiene article to a garment of the user. The inner surface of the elongated sheet is in contact with the release liner which is permanently or releasably attached to the inner surface of the packaging sheet. The release liner is preferably treated with a silicone-coating on the surface facing the disposable hygiene article so that it can be easily detached from the adhesive regions provided on the garment-facing surface of the disposable hygiene article.

The release liner may have a shape that is substantially the same as the shape of the disposable hygiene article inside the packaging unit or may have a shape that is different from the shape of the disposable hygiene article, as long as it can protect and cover the adhesive regions provided on the garment-facing surface of the disposable hygiene article. Thus, the size and shape of the release liner may vary, but suitably correspond to at least the size and shape which is sufficient to cover outer boundaries of the adhesive regions provided on the garment-facing surface of the disposable hygiene article. Suitably, the longitudinal extension of the release liner is 60-95% of the longitudinal extension of the packaging sheet and the transverse extension may be from 30-90% of the transverse extension of the packaging sheet.

The release liner may be manufactured from any suitable material known in the art, such as paper, non-woven, plastic film material and can be a single ply material or a laminate. The release liner may comprise a printed indicia, text, colour or combination thereof, and can be arranged on either sheet facing or disposable hygiene article facing surface of the release liner.

The release liner may be attached to the inner surface of the packaging sheet by means of at least one adhesive region. The adhesive region is suitably placed symmetrically to attach the release liner in an even manner and the adhesive region can extend along the whole length of the release liner. Alternatively, there may be several adhesive regions, such as from 2-8, preferably 2-5, such as 3 adhesive regions that are spaced apart, preferably equispaced, along the longitudinal direction of the release liner. Suitably, the release liner is permanently attached to the packaging sheet by means of a second adhesive. The second adhesive may be of any known type in the art, and can be for example a construction adhesive.

The disposable hygiene article is releasably affixed to the release liner on the disposable hygiene article facing surface of the release liner. Affixing may be obtained by using a releasable adhesive or by any other suitable fastening means known in the art, e.g. by means of hook-and-loop fastening means. The releasable adhesive to affix the disposable hygiene article to the release liner may be the same as used for the releasable coating strings on the packaging sheet, or it may another type of adhesive. Suitably another type of adhesive is used since the adhesive does not have to be resealable and needs to be suitable for use in contact with different garments without damaging the garment material.

The disposable hygiene article may be releasably attached to the release liner after the release liner has been attached to the inner surface of the packaging sheet, whereby the absorbent product is releasably attached to the packaging unit. Alternatively, the disposable hygiene article can be first releasably attached to the release liner and subsequently the release liner with the absorbent product is attached to the packaging sheet.

Further features of the invention are now described with reference to the appended drawings which are herein submitted to provide an example of a packaging unit but should not be regarded as limiting the scope of the invention in any way.

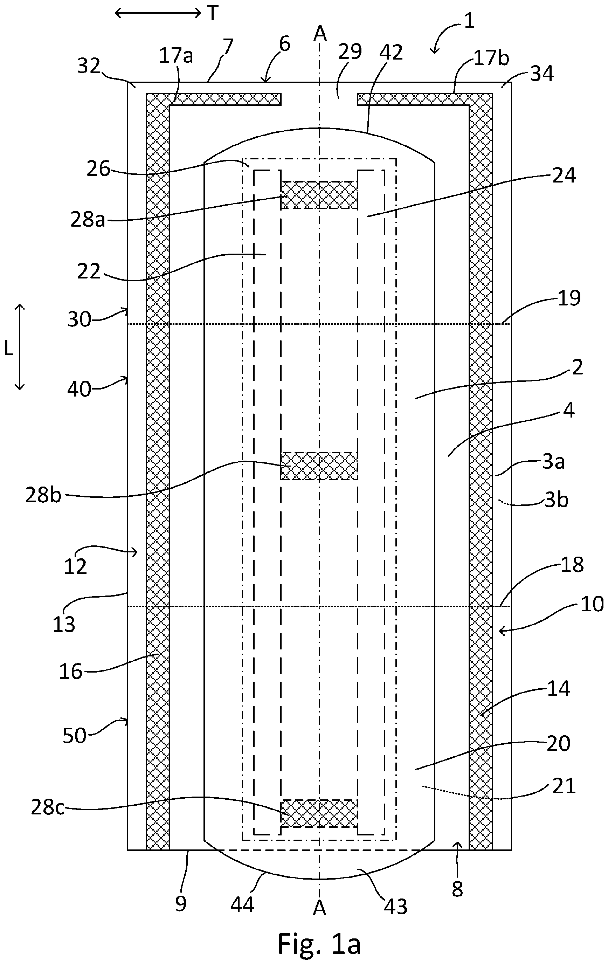

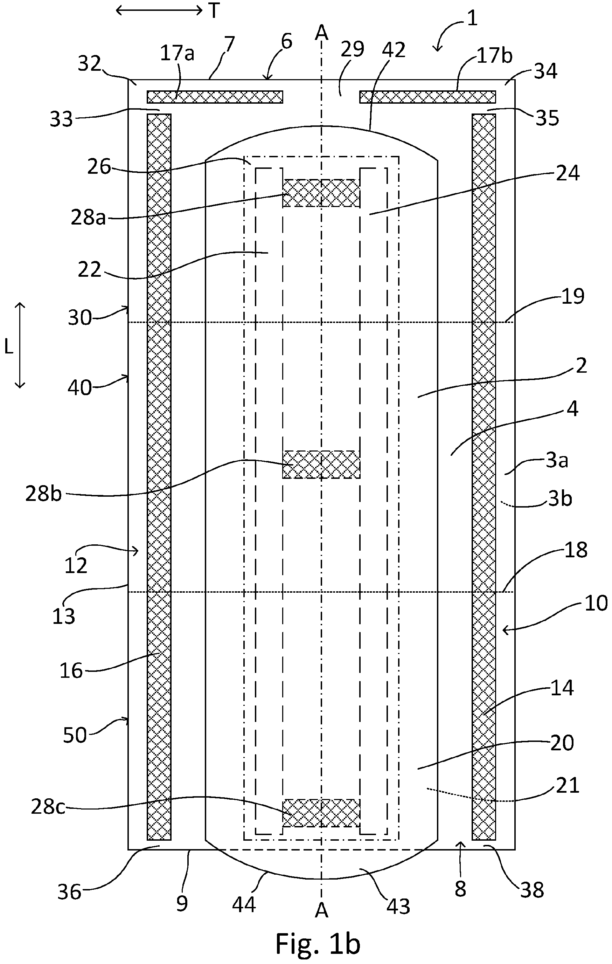

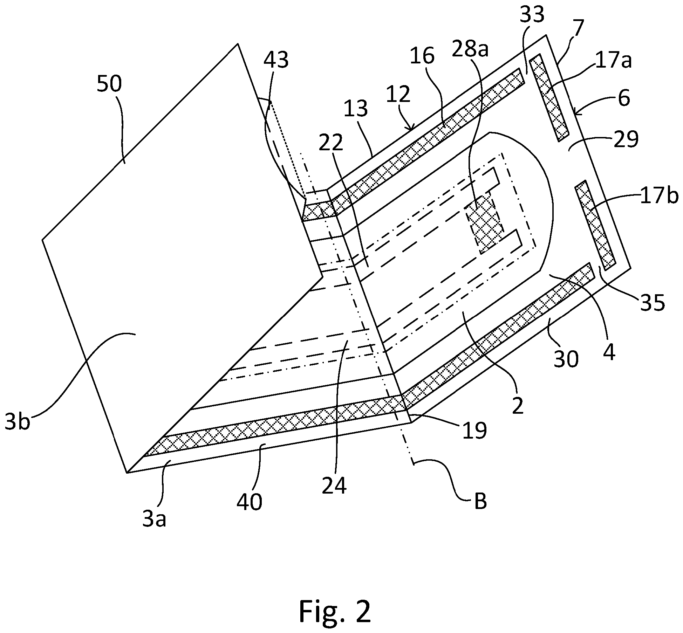

Fig. 1a shows a packaging unit according to one embodiment of the present invention from above. Fig. 1b shows a packaging unit according to another embodiment of the present invention from above and Fig. 2 shows the unit in partly folded in perspective view. Reference is equally made to all figures, but the differences between the embodiments in Fig 1a and 1b are indicated. Fig. 1a, Fig 1b and Fig. 2 thus show a packaging unit 1 comprising an elongated sheet 4 of packaging material having an inner surface 3a and an outer surface 3b. The sheet may be for example polyethylene-based film material and the basis weight of the sheet material may be from about 20 to 30 g/m², suitably from about 22-28 g/m². The elongated sheet 4 has a rectangular shape and the dimensions of the sheet can vary depending on the dimensions of a disposable hygiene article 2 placed on the sheet. The elongated sheet 4 comprises a first longitudinal end zone 6 that extends from the first end edge 7 of the sheet 4 inwards, i.e. towards the inside of the sheet 4. The end zone 6 includes a region for a coated resealable adhesive strings 17a, 17b. The first longitudinal end zone 6 includes the width of the resealable adhesive strings 17a, 17b, and a distance between the edge 7 of the sheet and the resealable adhesive strings 17a, 17b. The first longitudinal end zone 6 can extend inwards from the edge 7 of the sheet for example about 1.5-20 mm, and thus the end zone 6 has a width of from 1.5-20 mm, even though wider end zone could be possible. The packaging unit further comprises a second longitudinal end zone 8, which includes a second end edge 9 of the sheet 4 which may extend inwards from the second end edge 9 of the sheet 4 from 1.5 to 20 mm. The longitudinal end zones 6, 8 extend in a direction transverse (T) to a longitudinal direction (L) of the elongated sheet 4.

In Fig. 1a and 1b there are also two side edge zones 10 and 12 shown which extend in the longitudinal direction (L) of the elongated sheet 4. In a similar manner as the first end zone 6, the side edge zones 10, 12 extend from the respective edge 11, 13 of the sheet 4 inwards, i.e. towards the inside of the sheet 4. The first longitudinal end zone 6 and the side edge zones 10 and 12 include a respective region for coated resealable adhesive strings, 17a, 17b and 14, 16. For example, the side edge zones 10, 12 can extend inwards from the respective edge 11, 13 of the sheet 4 about 1.5-20 mm, and thus the side edge zones 10, 12 have a width of from 1.5-20 mm. The side edge zones could also be wider.

As mentioned, the side edge zones 10, 12 and the first longitudinal end zone 6 each comprise resealable adhesive coating strings 14, 16 and 17a, 17b that extend along the length of the side edge zones 10, 12 and the first longitudinal end zone 6 (strings 17a and 17b), respectively.

The length of the respective coated resealable adhesive string 14, 16 and 17a, 17b can be from 80-99% of the length of the respective zone. The side edge zones have a length in the longitudinal direction of the sheet and the first end zone has a length in the direction transverse (T) to the longitudinal direction (L) of the sheet.

The width of the respective coated resealable adhesive string 14, 16 and 17a, 17b in a direction inwards of the sheet may be for example from 1-15 mm, but other width dimensions could be possible.

As shown in Fig. 1a, 1b and 2, the resealable adhesive coating strings 14, 16 and 17a, 17b can be positioned such that there is an adhesive free edge area extending from the edge of the sheet 4 inwards and along the length of the side edge zones 10, 12 and the first longitudinal end zone 6, respectively. The adhesive free area may have a width that corresponds to the width of the adhesive strings 14, 16 and 17a, 17b of it may be smaller or larger. Suitably, the width of the adhesive free area is from 0.5-10 mm, but is not limited to that.

In Fig. 1b is shown an embodiment in which, in order to evacuate air from the packaging unit when it is folded, an adhesive free area or gap 33 and/or 35 is formed between the resealable adhesive coating strings 14, 16 and 17a, 17b extending along the side edge zones 10, 12 and the resealable adhesive coating string 17a or 17b extending along the first longitudinal edge zone 6 in a first corner area 32 and second corner area 34, respectively. Thus, the resealable adhesive string 14, 16 and 17a, 17b are positioned such that at least one of the corner areas 32, 34 of the packaging unit has an adhesive-free area or gap 33, 35, which can serve as an evacuation opening for air when the packaging unit is sealed. The gaps 33 and 35 are also avoiding the adhesive or glue overlapping between adhesive coating strings which could result in a need for excessive opening strength. There are also preferably adhesive free areas or gaps 36 and 38 between the edge 9 of the second end zone 8 and the resealable adhesive strings 14 and 16 extending along the side edge zones 10, 12. The gaps 36 and 38 guarantee that no adhesive or glue falls outside of the sheet 4 which would contaminate the machine elements. The adhesive free gaps 33, 35, 36 and 38 also facilitate the opening of the product.

In the embodiment shown in Fig 1a, there are no gaps in the corner areas 32, 34 between the resealable adhesive coating strings 17a and/or 17 b in the first longitudinal edge zone 6 and the resealable adhesive coating strings 14, 16 extending along the side edge zones 10, 12. Further, the resealable adhesive coating strings 14, 16 extending along the side edge zones 10, 12 extend all the way to the second end edge 9. In this way, a simple production process can be provided.

As shown in Fig 1a, 1b and 2, the packaging unit 1 further comprises an elongated disposable hygiene article 2 extending in a longitudinal direction (L) of the elongated sheet 4 and is placed on the inner surface 3a of the sheet 4 so that it fits within the area limited by the side edge zones 10, 12 and the first longitudinal end zone 6. It is important that the disposable hygiene article 2 does not extend inside the side edge zones 10, 12 and the first longitudinal end zone 6 so that it will not come into contact with the resealable adhesive coating strings 14, 16 and 17a, 17b. Further, the disposable hygiene article 2 is preferably placed on the sheet 4 such that a first edge 42 of the article (front parts) is adjacent to the first longitudinal end zone 6, and a second end edge 44 of the article (rear part) is adjacent to the second longitudinal end zone 8. The disposable hygiene article 2 is also placed at a distance from the resealable adhesive coating strings 17a, 17b. In the embodiment shown in Fig 1b, the second longitudinal end zone 8 is adhesive-free. In the embodiment shown in Fig 1a, the second longitudinal end zone 8 is adhesive-free between the resealable adhesive strings 14, 16 in the side edge zones 10,12.

The disposable hygiene article has a user facing side 20 and a garment facing side 21. The garment facing side is partially covered by adhesive regions 22 and 24 to which a release liner 26 is releasably attached. The purpose of the release liner 26 is to protect the adhesive regions 22 and 24 which are used to affix the disposable hygiene article 2 to a garment of a user. In the example embodiment shown in the figures the release liner 26 is attached to the inner surface 3a of the packaging sheet 4 by means of adhesive. As shown in Fig. 1a and 1b there are three centrally positioned and suitably equispaced adhesive regions 28a, 28b and 28c arranged to attach the release liner 26 to the packaging sheet 4. By centrally positioned is meant that the adhesive regions are at least partly located in the area of a central longitudinal axis (A-A).

As best visible in Fig 1a and 1b, the packaging unit 1 further comprises a first folding axis 18 and a second folding axis 19, each folding axis extending in a direction transverse to the longitudinal direction (L) of the elongated sheet 4. The first folding axis 18 is arranged between a third region 50 in the packaging unit 1 and a second region 40 in the packaging unit 1. The second folding axis 19 is arranged between the second region 40 and a first region of the packaging unit 30. The packaging unit 1 is formed by folding the elongated sheet 4, to which the disposable hygiene article is attached, along the first folding axis 18 and subsequently along the second folding axis 19.

As shown in Fig. 1a and 1b the release liner 26 is fastened to the inner surface 3a of the elongated packaging sheet 4 by means of the separate adhesive regions 28a, 28b and 28c, which are suitably placed in the first, second and third region, respectively. The disposable hygiene article could also be fastened directly to the elongated sheet by means of adhesive which is provided to be used as a fastener towards the garment of the user, such as a panty. (not shown). In that case the inner surface 3a of the elongated packaging sheet 4 is preferably coated with a silicone-based coating in the areas where the hygiene article is placed and wherein the areas where the resealable adhesive is coated are free of the silicone-based coating.

The first longitudinal end zone 6 is comprised in the first region 30. The first region 30 forms a lid for the packaging unit 1 after folding. The first longitudinal end zone 6 comprises at least two adjacent resealable adhesive coating strings 17a, 17b that extend along the length of the first longitudinal end zone 6. The resealable adhesive coating strings 17a, 17b are arranged such that a central adhesive free area 29 is formed in the first longitudinal end zone 6. This central adhesive free area 29 forms a central gap, which facilitates the opening of the packaging unit. The central adhesive free area 29 is suitably arranged symmetrically in relation to the central longitudinal axis (A-A), whereby equally distributed pulling forces acting on the resealable adhesive coating strings 14, 16 in the respective side edge zone 10, 12 can be obtained. The width of the adhesive free area 29 can be for example 10-50 mm, suitably 20-30 mm, so that a thumb of a normal user will fit into the opening to facilitate the opening of the product.

Reference is now made especially to Fig 1b and 2, which show an embodiment of the invention in which the elongated disposable hygiene article 2 extends in a longitudinal direction (L) of the elongated sheet 4 and is placed on the inner surface 3a of the sheet 4 such that it fits within the area limited by the side edge zones 10, 12 and the first longitudinal end zone 6, and has an end portion 43 having a predetermined length that extends beyond the second longitudinal end zone 8 in the longitudinal direction (L). The end portion 43 has preferably a rounded edge 44 and is shown in both Fig. 1a and 1b. The end portion 43 is preferably a rear portion of the disposable hygiene article 2. The unit 1 in Fig. 1b and 2 is arranged to be folded along the first folding axis 18 such that the third region 50 is pressed against the second region 40. At least part of the end portion 43 that extends beyond the second longitudinal end zone 8 is pressed against the first region 30, since the length of the second region in the longitudinal direction of the unit is shorter than the total length of the third region and the end portion 43. When folding the unit 1 to form a final product, the unit is further arranged to be folded along the second folding axis 19 such that the first region 30 together with the end portion 43 extending beyond the second longitudinal end zone 8 are pressed against the outer surface 21 of the elongated sheet 4 in the third region 50. In this way the end portion 43 forms a gripping portion when the unit is opened. The gripping portion 43 will also stand out when the unit is opened, since it is not attached to the outer surface 21 of the sheet in the first region 30. In another variant, the second folding axis 19 is arranged such that when the unit is folded the end portion 43 will not be pressed against the outer surface 21 of the elongated sheet 4 in the third region 50 and thus will not stand out. In any case the end portion 43 may be used a gripping portion. Also, since the end portion 43 will be positioned centrally and has preferably a rounded edge shape, it is easy to grip the end portion at a central location whereby the pulling forces will be equally distributed on both side edge zones and the resealable adhesive coating strings thereof when opening the unit 1. Thereby a neat opening of the packaging unit can be obtained. Thus, in addition to the the adhesive free area 29 forming a central gap in the lid, i.e. in the first region 30 of the packaging unit, the pulling forces can also be distributed equally when opening the folded packaging unit and the attachment between the third region 50 and the second region 40 by means of the end portion 43.

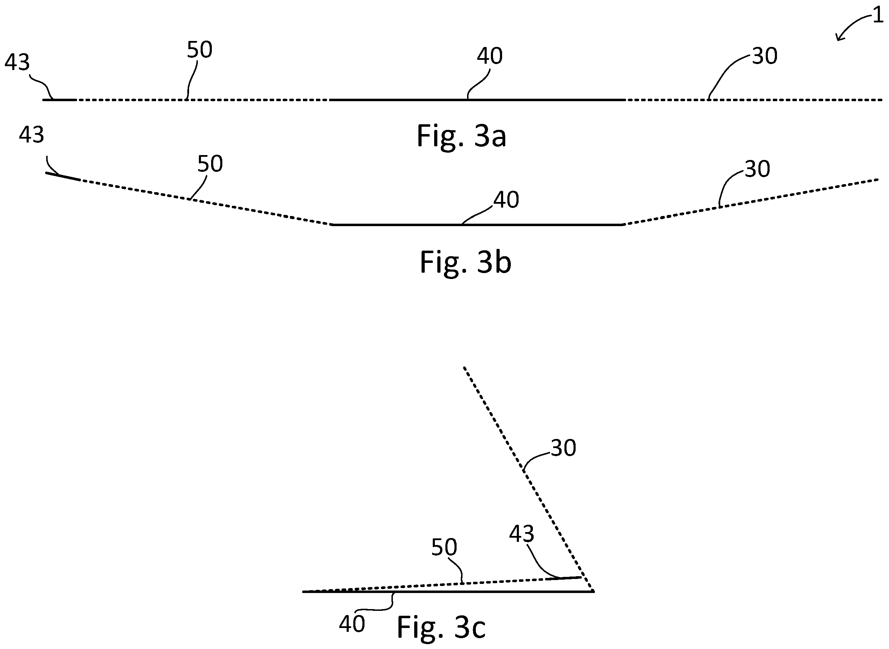

The folding procedure of the embodiment in which the end portion 43 is not pressed against the outer surface 21 of the elongated sheet 4 in the third region 50 is shown schematically in Fig 3a-3c. The packaging unit 1 is shown from the side and to distinguish the different regions 30, 40 and 50 and the end portion 43, the regions are illustrated with either dotted lines or continuous lines. In Fig. 3a the unit is shown in an unfolded state. Fig. 3b shows the different regions 30, 40 and 50 and the end portion 43 in a partially folded state to illustrate folding axis of the unit. In Fig. 3c it is shown that the third region 50 is folded towards the second region 40. It can be seen that the end portion 43 is in line with the third region 50

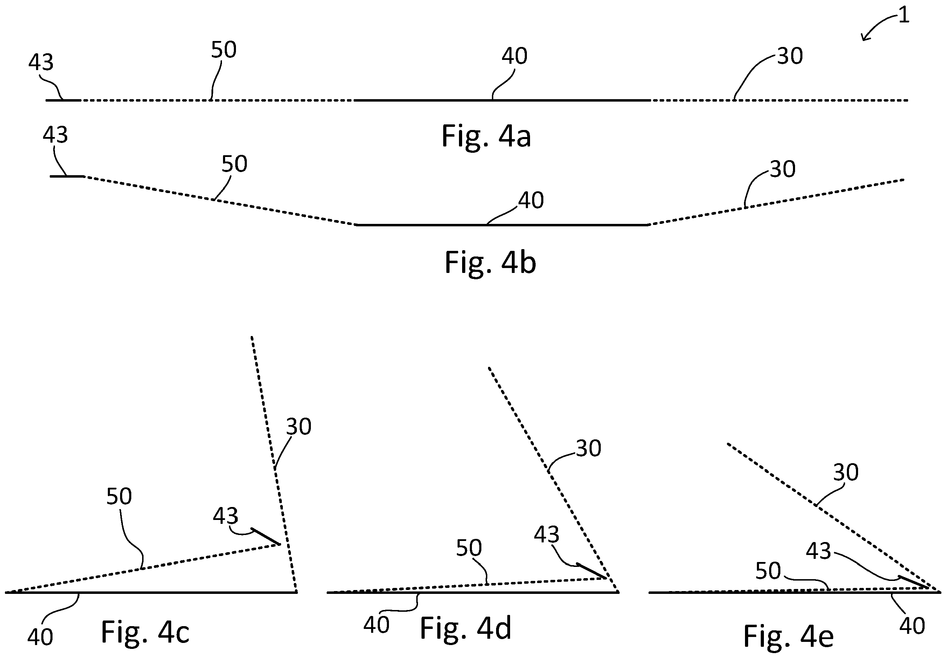

The folding procedure of the embodiment in which the end portion 43 is pressed against the outer surface 21 of the elongated sheet 4 in the third region 50 is shown schematically in Fig. 4a-4e. The packaging unit 1 is shown from the side and to distinguish the different regions 30, 40 and 50 and the end portion 43, the regions are illustrated with either dotted lines or continuous lines. In Fig. 4a the unit is shown in an unfolded state. Fig. 4b shows the different regions 30, 40 and 50 and the end portion 43 in a partially folded state to illustrate folding axis of the unit. In Fig. 4c it is shown that the third region 50 is folded towards the second region 40. It can be seen that the end portion 43 stands out since the total length of the third portion 50 and the end portion 43 is longer than the length of the second region. As can be seen, the end portion is easy to grip. In Fig. 4d and 4e a nearly completely folded unit before application of pressure is shown.

The present invention also relates to a method of forming a packaging unit 1 for disposable hygiene articles. The unit 1 comprises an elongated sheet of packaging material and a disposable hygiene article.

The method according to a first aspect comprises the steps of:
- providing an elongated sheet 4 of packaging material having an inner surface 3a and an outer surface 3b, a first longitudinal end zone 6 and a second longitudinal end zone 8, wherein the longitudinal end zones 6, 8 extend in a direction transverse (T) to a longitudinal direction (L) of the elongated sheet 4, and two side edge zones 10, 12 which extend in the longitudinal direction (L) of the elongated sheet 4,
- attaching an elongated disposable hygiene article 2 extending in the longitudinal direction (L) of the elongated sheet 4 and placing the article 2 on the inner surface 3a of the sheet 4 so that it fits within the area limited by the side edge zones 10, 12 and the first longitudinal end zone 6,
- coating resealable adhesive strings 14, 16, 17a, 17b along the lengths of the side edge zones 10, 12 and the first longitudinal end zone 6, and forming a central adhesive free area 29 between the adhesive strings 17a, 17b in the first longitudinal end zone 6,
- providing a first folding axis 18 and a second folding axis 19 in a direction transverse (T) to the longitudinal direction (L) of the unit 1 which divide the unit 1 into a first region 30, second region 40 and a third region 50,
- folding the elongated sheet 4 with the disposable hygiene article 2 along the first folding axis 18 inwards, such that the inner surface 3a of the elongated sheet 4 and the resealable adhesive coating strings 14, 16 in the third region 50 and the second region 40 are pressed against each other,
- folding the elongated sheet 4 with the disposable hygiene article 2 along a second folding axis 19 inwards, such that the outer surface 3b of the sheet at the third region 50 is pressed against the resealable adhesive coating strings 14, 16, 17a and 17b in the first region 30 and the inner surface 3a of the sheet 4 at the first region 30, and
- closing the packaging unit by applying pressure.

In the method when folding the elongated sheet 4 of the packaging material and the disposable hygiene article 2 along the first folding axis 19 inwards such that the inner surface 3a of the elongated sheet 4 and the resealable adhesive coating strings 14, 16 in the side edge zones 10, 12 are pressed against each other, and the third region 50 is pressed against the outer surface 3b of the sheet 4 in the second region 40 the end portion 43, or at least part of the end portion 43, is preferably pressed against the first region. Further, when folding the elongated sheet 4 with the disposable hygiene article 2 along a second folding axis 19 inwards, such that the outer surface 3b of the sheet in the third region 50 is pressed against the resealable adhesive coating strings 14, 16, 17a and 17b in the first region 30 and the inner surface 3a of the sheet 4 in the first region 30, the end portion 43, or at least part of the end portion 43, is pressed towards the first folding axis 18 and therefore forms gripping portion when the unit is opened.

The method may further comprise attaching a release liner to the disposable hygiene article 2 by means of adhesive regions 22, 24, and by attaching the disposable hygiene article 2 including the release liner 26 to the elongated sheet by means of adhesive regions 28a, 28b and 28c located in the first 30, second 40 and third region 50, respectively.

It should be noted that when the packaging unit according to the present invention is used for disposal, the user can choose to roll the packaging unit and the soiled article positioned on it rather than folding it.

Although the present invention has been described with reference to various embodiments, those skilled in the art will recognise that changes may be made without departing from the scope of the invention. It is intended that the detailed description be regarded as illustrative and that the appended claims including all the equivalents are intended to define the scope of the invention.

## Claims

1. A packaging unit (1) for a disposable hygiene article (2), the unit comprising
- an elongated sheet (4) of packaging material having an inner surface (3a) and an outer surface (3b) and the elongated sheet comprising:
- a first longitudinal end zone (6) and a second longitudinal end zone (8), wherein the longitudinal end zones extend in a direction transverse (T) to a longitudinal direction (L) of the elongated sheet 4, and
- two side edge zones (10, 12) which extend in the longitudinal direction (L) of the elongated sheet (4), and
- the side edge zones (10, 12) and the first longitudinal end zone (6) comprise a resealable adhesive coating string (14; 16; 17a, 17b) along the length of the side edge zones (10, 12) and the first longitudinal end zone (6), respectively, and wherein
- the unit (1) further comprises an elongated disposable hygiene article (2) extending in a longitudinal direction (L) of the elongated sheet (4) placed on the inner surface (3a) of the sheet (4) such that it fits within an area limited by the side edge zones (10, 12) and the first longitudinal end zone (6),
the unit (1) being formed by folding the elongated sheet (4) and the disposable hygiene article (2), the unit having a first folding axis (18) and a second folding axis (19), each folding axis extending in a direction transverse (T) to the longitudinal direction (L) of the elongated sheet 4, and the first folding axis (18) is arranged between a third region (50) in the packaging unit and a second region (40) in the packaging unit and the second folding axis (19) is arranged between the second region (40) and a first region (30) of the packaging unit (1) and wherein the first region (30) is arranged to form a lid for the packaging unit after folding, **characterized in that** the first longitudinal end zone (6) is comprised in the first region (30) and comprises at least two adjacent, in the direction transverse to the longitudinal direction of the elongated sheet, resealable adhesive coating strings (17a, 17b) that extend along the length of the first longitudinal end zone (6) and the strings (17a, 17b) being arranged such that a central adhesive free area (29) is formed to form a gripping gap.

2. The packaging unit according to claim 1, wherein the disposable hygiene article (2) is placed on the inner surface (3a) of the sheet (4) at a predetermined distance from the resealable adhesive coating strings (17a, 17b) in the first longitudinal end zone (6), in the direction of a central axis (A) of the unit (1).

3. The packaging unit according to claim 1 or 2, wherein the article (2) has an end portion (43) having a pre-determined length that extends beyond the second longitudinal end zone (8).

4. The packaging unit according to claim 3, wherein the unit (1) is arranged to be folded along a first folding axis (18) such that the third region (50) is pressed against the second region (40) and the end portion (43) that extends beyond the second longitudinal end zone (8) is pressed against the first region (30), and the unit (1) is further arranged to be folded along a second folding axis (19) such that the first region (30) together with the end portion (43) are pressed against the outer surface (3b) of the elongated sheet (4) in the third region (50), whereby the end portion (43) forms a gripping portion when the unit (1) is opened.

5. The packaging unit (1) according to any one of claims 1 to 4, wherein the end portion (43) comprises a second end edge (44) of the article (2), which second end edge (44) has a rounded shape.

6. The packaging unit (1) according to any one of the preceding claims, wherein the disposable hygiene article (2) comprises a release liner (26) releasably fastened to a garment facing side (21) of the article (2) by means of an adhesive and wherein the release liner (26) is fastened to the inner surface (3a) of the elongated sheet (4) by means of adhesive.

7. The packaging unit (1) according to claim 6, wherein the release liner (26) is fastened to the inner surface (3a) of the elongated sheet (4) by means of separate adhesive regions (28a; 28b, 28c) placed in the first (30), second (40) and third region (50), respectively.

8. The packaging unit (1) according to any one of the preceding claims 1-5, wherein the disposable hygiene article (2) is fastened directly to the inner surface (3a) of the elongated sheet (4) by means of adhesive.

9. The packaging unit (1) according to claim 8, wherein the inner surface (3a) of the elongated sheet (4) is coated with a silicone-based coating in the areas where the hygiene article (2) is placed and wherein the areas where the resealable adhesive is coated as resealable adhesive coating strings (14; 16; 17a, 17b) are free of the silicone-based coating.

10. A packaging unit (1) according to any one of the preceding claims, wherein the elongated sheet (4) has a rectangular shape.

11. The packaging unit (1) according to any one of the preceding claims, wherein the elongated sheet of packaging material is a plastic film, such as a polyethylene film, nonwoven or plastic laminate having a basis weight of 20-30 g/m².

12. The packaging unit according to any one of the preceding claims, wherein the second longitudinal end zone (8) between the resealable adhesive coating strings (14, 16) in the side edge zones (10, 12) is adhesive-free.

13. The packaging unit (1) according to any one of the preceding claims, wherein an adhesive free gap (33; 35; 36; 38) is formed between the resealable adhesive coating strings (14, 16) extending along the side edge zones (10, 12) of the sheet (4) and the resealable adhesive coating strings (17a, 17b) extending along the first longitudinal edge zone (6).

14. The packaging unit according to any one of the preceding claims, wherein the resealable adhesive is a pressure sensitive hotmelt adhesive.

15. The packaging unit according to any one of the preceding claims, wherein said elongated sheet (4) is opaque and/or comprises print.

16. Method of forming a packaging unit (1) for a disposable hygiene article (2), comprising the steps of:
- providing an elongated sheet (4) of packaging material having an inner surface (3a) and an outer surface (3b), a first longitudinal end zone (6) and a second longitudinal end zone (8), wherein the longitudinal end zones (6, 8) extend in a direction transverse (T) to a longitudinal direction (L) of the elongated sheet 4, and two side edge zones (10, 12) which extend in the longitudinal direction (L) of the elongated sheet (4),
- attaching an elongated disposable hygiene article (2) extending in the longitudinal direction (L) of the elongated sheet (4) and placing the article (2) on the inner surface (3a) of the sheet (4) so that it fits within the area limited by the side edge zones (10, 12) and the first longitudinal end zone (6),
- coating resealable adhesive strings (14, 16, 17a, 17b) along the length of each of the side edge zones (10, 12) and the first longitudinal end zone (6), and forming a central adhesive free area (29) between the resealable adhesive coating strings (17a, 17b) in the first longitudinal end zone (6) to form a gripping gap,
- providing a first folding axis (18) and a second folding axis (19) in a direction transverse (T) to the longitudinal direction (L) of the unit (1) which divide the unit (1) into a first region (30), second region (40) and a third region (50),
- folding the elongated sheet (4) with the disposable hygiene article (2) along the first folding axis (18) inwards, such that the inner surface (3a) of the elongated sheet (4) and the resealable adhesive coating strings (14, 16) extending along the side edge zones (10, 12) in the third region (50) and the second region (40) are pressed against each other,
- folding the elongated sheet (4) with the disposable hygiene article (2) along the second folding axis (19) inwards, such that the outer surface (3b) of the sheet (4) in the third region (50) is pressed against the resealable adhesive coating strings (14; 16; 17a, 17b) in the first region (30) and the inner surface (3a) of the sheet (4) in the first region (30), and
- closing the packaging unit (1) by applying pressure.

17. Method of forming a packaging unit for disposable hygiene articles according to claim 16, wherein when
- folding the elongated sheet (4) of the packaging material and the disposable hygiene article (2) along the first folding axis (19) inwards such that the inner surface (3a) of the elongated sheet (4) and the resealable adhesive coating strings (14, 16) in the side edge zones (10, 12) are pressed against each other, and when the third region (50) is pressed against the outer surface (3b) of the sheet (4) in the second region (40), an end portion (43) extending beyond the second end zone (8) is pressed against the first region (30), and when folding the elongated sheet (4) with the disposable hygiene article (2) along a second folding axis (19) inwards such that the outer surface (3b) of the sheet (4) in the third region (50) is pressed against the resealable adhesive coating strings (14; 16; 17a and 17b) in the first region (30) and the inner surface (3a) of the sheet (4) in the first region (30), the end portion (43) is pressed towards the first folding axis (18) and therefore forms gripping portion when the unit is opened.

18. Method according to any one of claims 15 or 16, further comprising attaching a release liner to the disposable hygiene article (2) by means of adhesive regions (22, 24) and by attaching the disposable hygiene article (2) to the elongated sheet by means of adhesive regions (28a, 28b and 28c) located in the first (30), second (40) and third region (50), respectively.

## Patentansprüche

1. Verpackungseinheit (1) für einen Einweg-Hygieneartikel (2), wobei die Einheit Folgendes aufweist
- einen langgestreckten Bogen (4) aus Verpackungsmaterial mit einer Innenfläche (3a) und einer Außenfläche (3b), wobei der langgestreckte Bogen Folgendes aufweist:
- eine erste Längsendzone (6) und eine zweite Längsendzone (8), wobei sich die Längsendzonen in einer Richtung quer (T) zu einer Längsrichtung (L) des langgestreckten Bogens (4) erstrecken, und
- zwei Seitenkantenzonen (10, 12), die sich in der Längsrichtung (L) des langgestreckten Bogens (4) erstrecken, und
- wobei die Seitenkantenzonen (10, 12) und die erste Längsendzone (6) einen wiederverschließbaren Klebstoffbeschichtungsstrang (14; 16; 17a, 17b) entlang der Länge der Seitenkantenzonen (10, 12) bzw. der ersten Längsendzone (6) aufweisen, und
- wobei die Einheit (1) ferner einen langgestreckten Einweg-Hygieneartikel (2) aufweist, der sich in einer Längsrichtung (L) des langgestreckten Bogens (4) erstreckt und auf der Innenfläche (3a) des Bogens (4) so angeordnet ist, dass er in einen Bereich passt, der durch die Seitenkantenzonen (10, 12) und die erste Längsendzone (6) begrenzt ist,
wobei die Einheit (1) durch Falten des langgestreckten Bogens (4) und des Einweg-Hygieneartikels (2) gebildet wird, wobei die Einheit eine erste Faltachse (18) und eine zweite Faltachse (19) aufweist, wobei sich jede Faltachse in einer Richtung quer (T) zur Längsrichtung (L) des langgestreckten Bogens (4) erstreckt, und die erste Faltachse (18) zwischen einem dritten Bereich (50) in der Verpackungseinheit und einem zweiten Bereich (40) in der Verpackungseinheit angeordnet ist und die zweite Faltachse (19) zwischen dem zweiten Bereich (40) und einem ersten Bereich (30) der Verpackungseinheit (1) angeordnet ist und wobei der erste Bereich (30) angeordnet ist, um nach dem Falten einen Deckel für die Verpackungseinheit zu bilden, **dadurch gekennzeichnet, dass** die erste Längsendzone (6) in dem ersten Bereich (30) enthalten ist und mindestens zwei benachbarte, in der Richtung quer zur Längsrichtung des langgestreckten Bogens wiederverschließbare Klebstoffbeschichtungsstränge (17a, 17b) aufweist, die sich entlang der Länge der ersten Längsendzone (6) erstrecken, und die Stränge (17a, 17b) so angeordnet sind, dass ein zentraler klebstofffreier Bereich (29) gebildet wird, um einen Greifspalt zu bilden.

2. Verpackungseinheit nach Anspruch 1, wobei der Einweg-Hygieneartikel (2) auf der Innenfläche (3a) des Bogens (4) in einem vorbestimmten Abstand von den wiederverschließbaren Klebstoffbeschichtungssträngen (17a, 17b) in der ersten Längsend-Zone (6) in Richtung einer Mittelachse (A) der Einheit (1) angeordnet ist.

3. Verpackungseinheit nach Anspruch 1 oder 2, wobei der Artikel (2) einen Endabschnitt (43) mit einer vorbestimmten Länge aufweist, der sich über die zweite Längsendzone (8) hinaus erstreckt.

4. Verpackungseinheit nach Anspruch 3, wobei die Einheit (1) so angeordnet ist, dass sie entlang einer ersten Faltachse (18) so gefaltet werden kann, dass der dritte Bereich (50) gegen den zweiten Bereich (40) gedrückt wird und der Endabschnitt (43), der sich über die zweite Längsendzone (8) hinaus erstreckt, gegen den ersten Bereich (30) gedrückt wird, und wobei die Einheit (1) ferner so angeordnet ist, dass sie entlang einer zweiten Faltachse (19) gefaltet werden kann, so dass der erste Bereich (30) zusammen mit dem Endabschnitt (43) gegen die Außenfläche (3b) des langgestreckten Bogens (4) im dritten Bereich (50) gepresst wird, wodurch der Endabschnitt (43) einen Greifabschnitt bildet, wenn die Einheit (1) geöffnet wird.

5. Verpackungseinheit (1) nach einem der Ansprüche 1 bis 4, wobei der Endabschnitt (43) eine zweite Endkante (44) des Artikels (2) aufweist, wobei die zweite Endkante (44) eine abgerundete Form hat.

6. Verpackungseinheit (1) nach einem der vorhergehenden Ansprüche, wobei der Einweg-Hygieneartikel (2) eine Schutzfolie (26) aufweist, die mittels eines Klebstoffs lösbar an einer der Kleidung zugewandten Seite (21) des Artikels (2) angebracht ist, und wobei die Schutzfolie (26) mittels eines Klebstoffs an der Innenfläche (3a) des langgestreckten Bogens (4) angebracht ist.

7. Verpackungseinheit (1) nach Anspruch 6, wobei die Schutzfolie (26) an der Innenfläche (3a) des langgestreckten Bogens (4) mittels separater Klebebereiche (28a; 28b, 28c), die jeweils im ersten (30), zweiten (40) und dritten Bereich (50) angeordnet sind, befestigt ist.

8. Verpackungseinheit (1) nach einem der vorhergehenden Ansprüche 1-5, wobei der Einweg-Hygieneartikel (2) direkt an der Innenfläche (3a) des langgestreckten Bogens (4) mittels Klebstoff befestigt ist.

9. Verpackungseinheit (1) nach Anspruch 8, wobei die Innenfläche (3a) des langgestreckten Bogens (4) in den Bereichen, in denen der Hygieneartikel (2) angeordnet ist, mit einer Beschichtung auf Silikonbasis beschichtet ist, und wobei die Bereiche, in denen der wiederverschließbare Klebstoff als wiederverschließbare Klebstoffbeschichtungsstränge (14; 16; 17a, 17b) beschichtet ist, frei von der Beschichtung auf Silikonbasis sind.

10. Verpackungseinheit (1) nach einem der vorhergehenden Ansprüche, wobei das langgestreckte Bogen (4) eine rechteckige Form hat.

11. Verpackungseinheit (1) nach einem der vorhergehenden Ansprüche, wobei der langgestreckte Bogen des Verpackungsmaterials eine Kunststofffolie, wie beispielsweise eine Polyethylenfolie, ein Vliesstofflaminat oder ein Kunststofflaminat mit einem Flächengewicht von 20-30 g/m² ist.

12. Verpackungseinheit nach einem der vorhergehenden Ansprüche, wobei die zweite Längsendzone (8) zwischen den wiederverschließbaren Klebstoffbeschichtungssträngen (14, 16) in den seitlichen Randzonen (10, 12) frei von Klebstoff ist.

13. Verpackungseinheit (1) nach einem der vorhergehenden Ansprüche, wobei ein klebstofffreier Spalt (33; 35; 36; 38) zwischen den wiederverschließbaren Klebstoffbeschichtungssträngen (14, 16), die sich entlang der Seitenkantenzonen (10, 12) des Bogens (4) erstrecken, und den wiederverschließbaren Klebstoffbeschichtungssträngen (17a, 17b), die sich entlang der ersten Längskantenzone (6) erstrecken, gebildet ist.

14. Verpackungseinheit nach einem der vorhergehenden Ansprüche, wobei der wiederverschließbare Klebstoff ein druckempfindlicher Heißschmelzklebstoff ist.

15. Verpackungseinheit nach einem der vorhergehenden Ansprüche, wobei der langgestreckte Bogen (4) undurchsichtig ist und/oder bedruckt ist.

16. Verfahren zur Bildung einer Verpackungseinheit (1) für einen Einweg-Hygieneartikel (2), das die folgenden Schritte aufweist:
- Bereitstellen eines langgestreckten Bogens (4) aus Verpackungsmaterial mit einer Innenfläche (3a) und einer Außenfläche (3b), einer ersten Längsendzone (6) und einer zweiten Längsendzone (8), wobei sich die Längsendzonen (6, 8) in einer Richtung quer (T) zu einer Längsrichtung (L) des langgestreckten Bogens (4) erstrecken, und zwei Seitenkantenzonen (10, 12), die sich in der Längsrichtung (L) des langgestreckten Bogens (4) erstrecken,
- Befestigen eines langgestreckten Einweg-Hygieneartikels (2), der sich in Längsrichtung (L) des langgestreckten Bogens (4) erstreckt, und Anordnen des Artikels (2) auf der Innenfläche (3a) des Bogens (4), so dass er in den durch die seitlichen Randzonen (10, 12) und die erste Längsend-Zone (6) begrenzten Bereich passt,
- Beschichten wiederverschließbarer Klebstoffstränge (14, 16, 17a, 17b) entlang der Länge jeder der Seitenkantenzonen (10, 12) und der ersten Längsendzone (6) und Bilden eines zentralen klebstofffreien Bereichs (29) zwischen den wiederverschließbaren Klebstoffbeschichtungssträngen (17a, 17b) in der ersten Längsendzone (6), um einen Greifspalt zu bilden,
- Vorsehen einer ersten Faltachse (18) und einer zweiten Faltachse (19) in einer Richtung quer (T) zur Längsrichtung (L) der Einheit (1), welche die Einheit (1) in einen ersten Bereich (30), einen zweiten Bereich (40) und einen dritten Bereich (50) teilen,
- Falten des langgestreckten Bogens (4) mit dem Einweg-Hygieneartikel (2) entlang der ersten Faltachse (18) nach innen, so dass die Innenfläche (3a) des langgestreckten Bogens (4) und die entlang der seitlichen Randzonen (10, 12) im dritten Bereich (50) und im zweiten Bereich (40) verlaufenden wiederverschließbaren Klebstoffbeschichtungsstränge (14, 16) gegeneinander gedrückt werden,
- Falten des langgestreckten Bogens (4) mit dem Einweg-Hygieneartikel (2) entlang der zweiten Faltachse (19) nach innen, so dass die Außenfläche (3b) des Bogens (4) im dritten Bereich (50) gegen die wiederverschließbaren Klebstoffbeschichtungsstränge (14; 16; 17a, 17b) im ersten Bereich (30) und die Innenfläche (3a) des Bogens (4) im ersten Bereich (30) gedrückt wird, und
- Schließen der Verpackungseinheit (1) durch Anwenden von Druck.

17. Verfahren zur Bildung einer Verpackungseinheit für Einweg-Hygieneartikel nach Anspruch 16, wobei, wenn
- der langgestreckte Bogen (4) des Verpackungsmaterials und der Einweg-Hygieneartikel (2) entlang der ersten Faltachse (19) nach innen gefaltet werden, so dass die Innenfläche (3a) des langgestreckten Bogens (4) und die wiederverschließbaren Klebstoffbeschichtungsstränge (14, 16) in den Seitenkantenzonen (10, 12) gegeneinander gedrückt werden, und wenn der dritte Bereich (50) gegen die Außenfläche (3b) des Bogens (4) im zweiten Bereich (40) gedrückt wird, ein Endabschnitt (43), der sich über die zweite Endzone (8) hinaus erstreckt, gegen den ersten Bereich (30) gedrückt wird, und wenn der langgestreckten Bogen (4) mit dem Einweg-Hygieneartikel (2) entlang einer zweiten Faltachse (19) nach innen gefaltet wird, so dass die Außenfläche (3b) des Bogens (4) im dritten Bereich (50) gegen die wiederverschließbaren Klebstoffbeschichtungsstränge (14, 16; 17a und 17b) in dem ersten Bereich (30) und die Innenfläche (3a) des Bogens (4) in dem ersten Bereich (30) gedrückt wird, der Endabschnitt (43) in Richtung der ersten Faltachse (18) gedrückt wird und daher einen Greifabschnitt bildet, wenn die Einheit geöffnet wird.

18. Verfahren nach einem der Ansprüche 15 oder 16, wobei das Verfahren weiterhin das Anbringen einer Schutzfolie an dem Einweg-Hygieneartikel (2) mittels Klebebereichen (22, 24) und das Anbringen des Einweg-Hygieneartikels (2) an dem langgestreckten Bogen mittels Klebebereichen (28a, 28b und 28c) aufweist, die sich in dem ersten (30), zweiten (40) bzw. dritten Bereich (50) befinden.

## Revendications

1. Unité d'emballage (1) pour un article d'hygiène jetable (2), l'unité comprenant
- une feuille allongée (4) de matériau d'emballage ayant une surface intérieure (3a) et une surface extérieure (3b) et la feuille allongée comprenant :
- une première zone d'extrémité longitudinale (6) et une seconde zone d'extrémité longitudinale (8), dans laquelle les zones d'extrémité longitudinales s'étendent dans une direction transversale (T) par rapport à une direction longitudinale (L) de la feuille allongée (4), et
- deux zones de bord latérales (10, 12) qui s'étendent dans la direction longitudinale (L) de la feuille allongée (4), et
- les zones de bord latérales (10, 12) et la première zone d'extrémité longitudinale (6) comprennent un cordon de revêtement adhésif refermable (14 ; 16 ; 17a, 17b) sur la longueur des zones de bord latérales (10, 12) et la première zone d'extrémité longitudinale (6), respectivement, et dans laquelle
- l'unité (1) comprend en outre un article d'hygiène jetable allongé (2) s'étendant dans une direction longitudinale (L) de la feuille allongée (4) placé sur la surface intérieure (3a) de la feuille (4) de telle sorte qu'il s'insère à l'intérieur une zone délimitée par les zones de bord latérales (10, 12) et la première zone d'extrémité longitudinale (6),
l'unité (1) étant formée en pliant la feuille allongée (4) et l'article d'hygiène jetable (2), l'unité ayant un premier axe de pliage (18) et un second axe de pliage (19), chaque axe de pliage s'étendant dans une direction transversale (T) par rapport à la direction longitudinale (L) de la feuille allongée (4), et le premier axe de pliage (18) est agencé entre une troisième région (50) dans l'unité d'emballage et une deuxième région (40) dans l'unité d'emballage et le second axe de pliage (19) est agencé entre la deuxième région (40) et une première région (30) de l'unité d'emballage (1) et dans laquelle la première région (30) est agencée pour former un couvercle pour l'unité d'emballage après pliage, **caractérisée en ce que** la première zone d'extrémité longitudinale (6) est comprise dans la première région (30) et comprend au moins deux cordons de revêtement adhésif refermable (17a, 17b) adjacents dans la direction transversale à la direction longitudinale de la feuille allongée qui s'étendent le long de la longueur de la première zone d'extrémité longitudinale (6) et les cordons (17a, 17b) étant agencés de telle sorte qu'une zone centrale sans adhésif (29) est formée pour former un espace de préhension.

2. Unité d'emballage selon la revendication 1, dans laquelle l'article d'hygiène jetable (2) est placé sur la surface intérieure (3a) de la feuille (4) à une distance prédéterminée des cordons de revêtement adhésif refermable (17a, 17b) dans la première zone d'extrémité longitudinale (6), dans la direction d'un axe central (A) de l'unité (1).

3. Unité d'emballage selon la revendication 1 ou 2, dans laquelle l'article (2) a une partie d'extrémité (43) ayant une longueur prédéterminée qui s'étend au-delà de la seconde zone d'extrémité longitudinale (8).

4. Unité d'emballage selon la revendication 3, dans laquelle l'unité (1) est agencée pour être pliée le long d'un premier axe de pliage (18) de telle sorte que la troisième région (50) est pressée contre la deuxième région (40) et la partie d'extrémité (43) qui s'étend au-delà de la seconde zone d'extrémité longitudinale (8) est pressée contre la première région (30), et l'unité (1) est en outre agencée pour être pliée le long d'un second axe de pliage (19) de telle sorte que la première région (30) et la partie d'extrémité (43) sont pressées contre la surface extérieure (3b) de la feuille allongée (4) dans la troisième région (50), moyennant quoi la partie d'extrémité (43) forme une partie de préhension lorsque l'unité (1) est ouverte.

5. Unité d'emballage (1) selon l'une quelconque des revendications 1 à 4, dans laquelle la partie d'extrémité (43) comprend un second bord d'extrémité (44) de l'article (2), lequel second bord d'extrémité (44) a une forme arrondie.

6. Unité d'emballage (1) selon l'une quelconque des revendications précédentes, dans laquelle l'article d'hygiène jetable (2) comprend une doublure anti-adhésive (26) fixée de manière amovible à un côté faisant face au vêtement (21) de l'article (2) au moyen d'un adhésif et dans laquelle la doublure anti-adhésive (26) est fixée à la surface intérieure (3a) de la feuille allongée (4) au moyen d'un adhésif.

7. Unité d'emballage (1) selon la revendication 6, dans laquelle la doublure anti-adhésive (26) est fixée à la surface intérieure (3a) de la feuille allongée (4) au moyen de régions adhésives séparées (28a ; 28b, 28c) placées dans les première (30), deuxième (40) et troisième (50) régions, respectivement.

8. Unité d'emballage (1) selon l'une quelconque des revendications 1 à 5 précédentes, dans laquelle l'article d'hygiène jetable (2) est fixé directement à la surface intérieure (3a) de la feuille allongée (4) au moyen d'un adhésif.

9. Unité d'emballage (1) selon la revendication 8, dans laquelle la surface intérieure (3a) de la feuille allongée (4) est revêtue d'un revêtement à base de silicone dans les zones où l'article d'hygiène (2) est placé et dans laquelle les zones où l'adhésif refermable est appliqué en revêtement sous la forme d'un cordon de revêtement adhésif refermable (14 ; 16 ; 17a, 17b) sont exemptes du revêtement à base de silicone.

10. Unité d'emballage (1) selon l'une quelconque des revendications précédentes, dans laquelle la feuille allongée (4) a une forme rectangulaire.

11. Unité d'emballage (1) selon l'une quelconque des revendications précédentes, dans laquelle la feuille allongée de matériau d'emballage est un film plastique, tel qu'un film de polyéthylène, un non-tissé ou un stratifié en plastique ayant un poids de base de 20 à 30 g/m².

12. Unité d'emballage selon l'une quelconque des revendications précédentes, dans laquelle la seconde zone d'extrémité longitudinale (8) entre les cordons de revêtement adhésif refermable (14, 16) dans les zones de bord latérales (10, 12) est sans adhésif.

13. Unité d'emballage (1) selon l'une quelconque des revendications précédentes, dans laquelle un espace sans adhésif (33 ; 35 ; 36 ; 38) est formé entre les cordons de revêtement adhésif refermable (14, 16) s'étendant le long des zones de bord latérales (10, 12) de la feuille (4) et des cordons de revêtement adhésif refermable (17a, 17b) s'étendant le long de la première zone de bord longitudinale (6).

14. Unité d'emballage selon l'une quelconque des revendications précédentes, dans laquelle l'adhésif refermable est un adhésif thermofusible sensible à la pression.

15. Unité d'emballage selon l'une quelconque des revendications précédentes, dans laquelle ladite feuille allongée (4) est opaque et/ou comprend une impression.

16. Méthode de formation d'une unité d'emballage (1) pour un article d'hygiène jetable (2), comprenant les étapes consistant à :
- fournir une feuille allongée (4) de matériau d'emballage ayant une surface intérieure (3a) et une surface extérieure (3b), une première zone d'extrémité longitudinale (6) et une seconde zone d'extrémité longitudinale (8), dans laquelle les zones d'extrémité longitudinales (6, 8) s'étendent dans une direction transversale (T) par rapport à une direction longitudinale (L) de la feuille allongée 4, et deux zones de bord latérales (10, 12) qui s'étendent dans la direction longitudinale (L) de la feuille allongée (4),
- fixer un article d'hygiène allongé jetable (2) s'étendant dans la direction longitudinale (L) de la feuille allongée (4) et placer l'article (2) sur la surface intérieure (3a) de la feuille (4) de sorte qu'il s'ajuste dans la zone délimitée par les zones de bord latérales (10, 12) et la première zone d'extrémité longitudinale (6),
- appliquer en revêtement des cordons d'adhésif refermable (14, 16, 17a, 17b) le long de chacune des zones de bord latérales (10, 12) et de la première zone d'extrémité longitudinale (6), et former une zone centrale sans adhésif (29) entre les cordons de revêtement adhésif refermable (17a, 17b) dans la première zone d'extrémité longitudinale (6) pour former un espace de préhension,
- prévoir un premier axe de pliage (18) et un second axe de pliage (19) dans une direction transversale (T) par rapport à la direction longitudinale (L) de l'unité (1) qui divise l'unité (1) en une première région (30), une deuxième région (40) et une troisième région (50),
- plier la feuille allongée (4) avec l'article d'hygiène jetable (2) le long du premier axe de pliage (18) vers l'intérieur, de telle sorte que la surface intérieure (3a) de la feuille allongée (4) et les cordons de revêtement adhésif refermable (14, 16) s'étendant le long des zones de bord latérales (10, 12) dans la troisième région (50) et la deuxième région (40) sont pressées les uns contre les autres,
- plier la feuille allongée (4) avec l'article d'hygiène jetable (2) le long du second axe de pliage (19) vers l'intérieur, de sorte que la surface extérieure (3b) de la feuille (4) dans la troisième région (50) est pressée contre les cordons de revêtement adhésif refermable (14 ; 16 ; 17a, 17b) dans la première région (30) et la surface intérieure (3a) de la feuille (4) dans la première région (30), et
- fermer l'unité d'emballage (1) en appliquant une pression.

17. Méthode de formation d'une unité d'emballage pour des articles d'hygiène jetables selon la revendication 16, dans lequel lors du
- pliage de la feuille allongée (4) du matériau d'emballage et de l'article d'hygiène jetable (2) le long du premier axe de pliage (19) vers l'intérieur de telle sorte que la surface intérieure (3a) de la feuille allongée (4) et les cordons de revêtement adhésif refermable (14, 16) dans les zones de bord latérales (10, 12) soient pressés les uns contre les autres, et lorsque la troisième région (50) est pressée contre la surface extérieure (3b) de la feuille (4) dans la deuxième région (40), une partie d'extrémité (43) s'étendant au-delà de la seconde zone d'extrémité (8) est pressée contre la première région (30), et lors du pliage de la feuille allongée (4) avec l'article d'hygiène jetable (2) le long d'un second axe de pliage (19) vers l'intérieur de telle sorte que la surface extérieure (3b) de la feuille (4) dans la troisième région (50) soit pressée contre les cordons de revêtement adhésif refermable (14; 16; 17a et 17b) dans la première région (30) et la surface intérieure (3a) de la feuille (4) dans la première région (30), la partie d'extrémité (43) est pressée vers le premier axe de pliage (18) et forme donc une partie de préhension lorsque l'unité est ouverte.

18. Méthode selon l'une quelconque des revendications 15 ou 16, comprenant en outre la fixation d'une doublure anti-adhésive à l'article d'hygiène jetable (2) au moyen de régions adhésives (22, 24) et par fixation de l'article d'hygiène jetable (2) à la feuille allongée au moyen de régions adhésives (28a, 28b et 28c) situées dans les première (30), deuxième (40) et la troisième (50) régions, respectivement.
